## Europäisches Patentamt
## European Patent Office
## Office européen des brevets

(19)

(11) Veröffentlichungsnummer: **0 001 746 B2**

(12)

# NEUE EUROPÄISCHE PATENTSCHRIFT

(45) Veröffentlichungstag der neuen Patentschrift: **12.03.86**

(51) Int. Cl.⁴: **A 61 F 13/20**

(21) Anmeldenummer: **78100958.4**

(22) Anmeldetag: **22.09.78**

(54) Menstruationstampon und Verfahren zu seiner Herstellung.

(30) Priorität: **03.10.77 DE 2744466**

(43) Veröffentlichungstag der Anmeldung:
**16.05.79 Patentblatt 79/10**

(45) Bekanntmachung des Hinweises auf die Patenterteilung:
**29.04.81 Patentblatt 81/17**

(45) Bekanntmachung des Hinweises auf die Entscheidung
über den Einspruch:
**12.03.86 Patentblatt 86/11**

(84) Benannte Vertragsstaaten:
**BE CH FR GB NL SE**

(56) Entgegenhaltungen:
**CH - A - 565 553**
**DE - A - 1 642 146**
**DE - A - 2 424 439**
**DE - A - 2 559 606**
**DE - A - 2 636 899**
**DE - B - 1 079 796**
**DE - C - 827 838**
**GB - B - 527 827**
**US - A - 3 055 369**

(73) Patentinhaber: **Henkel Kommanditgesellschaft auf
Aktien, Postfach 1100 Henkelstrasse 67,
D-4000 Düsseldorf-Holthausen (DE)**

(72) Erfinder: **Wiegner, Georg, Breslauer Strasse 35,
D-4060 Viersen 11 (DE)**
Erfinder: **Schwolow, Hartmut, Friedensstrasse 24,
D-4155 Grefrath 2 (DE)**
Erfinder: **Klein, Dieter, Kölner Strasse 51,
D-5014 Erkelenz (DE)**
Erfinder: **Malaszkiewicz, Jürgen, Dr.,
Sonnbornstrasse 39, D-4000 Düsseldorf 12 (DE)**

EP 0 001 746 B2

ACTORUM AG

## Beschreibung

Die Erfindung betrifft einen Menstruationstampon, insbesondere Wickeltampon, aus beispielsweise aus Baumwolle oder Zellwolle bestehender Watte mit einer hydrophilen Beimischung verstärkter Saugfähigkeit, insbesondere mit einer im Hinblick auf Hydrophilie modifizierten Faserbeimischung.

Tampons bestehen in aller Regel aus Mischungen von Baumwolle und Zellwolle. Sie werden in unterschiedlichen Grössen, das heisst Wattemengen hergestellt und haben demzufolge auch unterschiedliche Saugleistungen. Entscheidend für die Qualität eines Menstruationstampons ist seine Saugfähigkeit.

Es gibt Stoffe, die in Beimischungen zur Watte die Saugleistung der Wattefaser wesentlich verbessern. Zu diesen Stoffen gehört beispielsweise carboxymethylierte Zellulose (CMC). Gleiche Effekte lassen sich auch mit anderen Stoffen, die besondere Saugeigenschaften haben, wie zum Beispiel Polymere oder geschäumte Polyurethane erreichen. Durch Einstreuen solcher Stoffe, zum Beispiel carboxymethylierter Zellulosefasern (CMC-Fasern) auf den Watteflor kann die Saugleistung des Gesamttampons nennenswert erhöht werden. So ergeben zum Beispiel 5%ige Beimischungen Verbesserungen der Saugkapazität von 15 bis 20%.

Ein Tampon mit einer im Hinblick auf Hydrophilie modifizierten Faserbeimischung ist beispielsweise aus der DE-A- 2 614 122 bekannt. Die Faserbeimischung ist hierbei über den ganzen Tampon gleichmässig verteilt. Der Tampon hat zwar eine sehr gute Saugfähigkeit, jedoch weist er eine Saugreserve bei plötzlichem Flüssigkeitsanfall nicht auf.

Zum Bilden solcher Zonen, die der Verbesserung der Sauggeschwindigkeit und Saugkapazität dienen mit dem Ziel, das Durchbluten am Tampon vorbei zu verhindern, wurden bisher verstärkte Wattezonen benutzt. Es zeigte sich jedoch, dass bei nennenswerter, zonenweiser Verstärkung der Wattemengen Überpressungen im Tampon unvermeidlich sind, wodurch wiederum die Saugfähigkeit stark reduziert wird.

Der Erfindung liegt die Aufgabe zugrunde, einen Tampon der eingangs genannten Art zu schaffen, der neben normaler guter Saugleistung eine Saugreserve zum Begegnen eines plötzlichen Flüssigkeitsanfalls aufweist. Die Lösung besteht darin, dass auf einem in bezug auf die Länge relativ kurzen Längsabschnitt des Tampons an dessen Ende, in dessen Mitte oder an dessen Kopf die Watte 2 bis 5% Beimischung enthält und die Watte der übrigen Bereiche des Tampons einen demgegenüber kleinen Anteil Beimischung enthält.

Dadurch, dass man erfindungsgemäss besonders saugfähige Stoffe durch gezieltes Aufbringen in einer schmalen Tamponzone inkorporiert, kommt es bei Anwendung zu deutlich sichtbaren Durchmesservergrösserungen und nennenswerten Saugvolumenverbesserungen im Bereich dieser besonders saugaktiven Zone. Weil es ferner gemäss der Erfindung genügt, dem saugaktiven Längsabschnitt des Tampons etwa 2 bis 5% der die Hydrophilie der Watte verbessernden Beimischung hinzuzufügen, können (die Saugfähigkeit reduzierende) Überpressungen im Tampon nicht auftreten. In der Regel kann es schliesslich günstig sein, zusätzlich die gesamte zum Herstellen des Tampons verwendete Watte mit einer geringen Menge der fraglichen Beimischung zu versetzen. Diese Menge soll jedoch klein sein gegenüber derjenigen, die in den als Saugreserve vorgesehenen saugaktiven Zonen enthalten ist.

Bei dem Verfahren zum Herstellen des erfindungsgemässen Wickeltampons geht man vorzugsweise so vor, dass längs einer Linie parallel zur Längsrichtung eines, insbesondere bereits kalandrierten und gegebenenfalls mit einer Grundlast der genannten Beimischung versetzten Wattebandes auf dieses ein gegenüber dem Watteband schmaler Streifen der die Hydrophilie verbessernden Beimischung aufgestreut wird, dass ferner die Beimischung durch Zusammenfalten der Ränder des Wattebandes eingeschlossen wird und dass schliesslich ein Kalandrieren oder Pressen des gefalteten Wattebandes erfolgt und gegebenenfalls nach einer Ablage in Rollen oder Hobbocks die Tampons aufgewickelt werden, wobei die Streifen der Beimischung mit Bandstärke übereinander geschichtet werden.

Anhand der schematischen Zeichnung werden weitere Einzelheiten der Erfindung erläutert; es zeigen:

Fig. 1 das Aufbringen der hydrophilen Beimischung auf ein Watteband,
Fig. 2 ein einfach gefaltetes Watteband,
Fig. 3 ein doppelt gefaltetes Watteband,
Fig. 4 ein Tampon beim Zusammenrollen und,
Fig. 5 bis 7 drei Tampons mit der erfindungsgemässen Saugzone am Ende, in der Mitte oder am Kopf.

Bei der Herstellung des erfindungsgemässen Menstruations-Wickeltampons wird zunächst die Wattefertigung über eine übliche Wattekrempel vorgenommen, die je nach Wunsch mit einer Grundlast der die Hydrophilie verbessernden und gegebenenfalls in den Watteflor eingestreuten Beimischung ausgerüstet sein kann. Dieser Wattekrempel nachgeschaltet folgt eine Kalandrierstation, die ein Watteband fertigt, das der doppelten Breite des später in der Tampon-Maschine befindlichen Wattebandes entspricht.

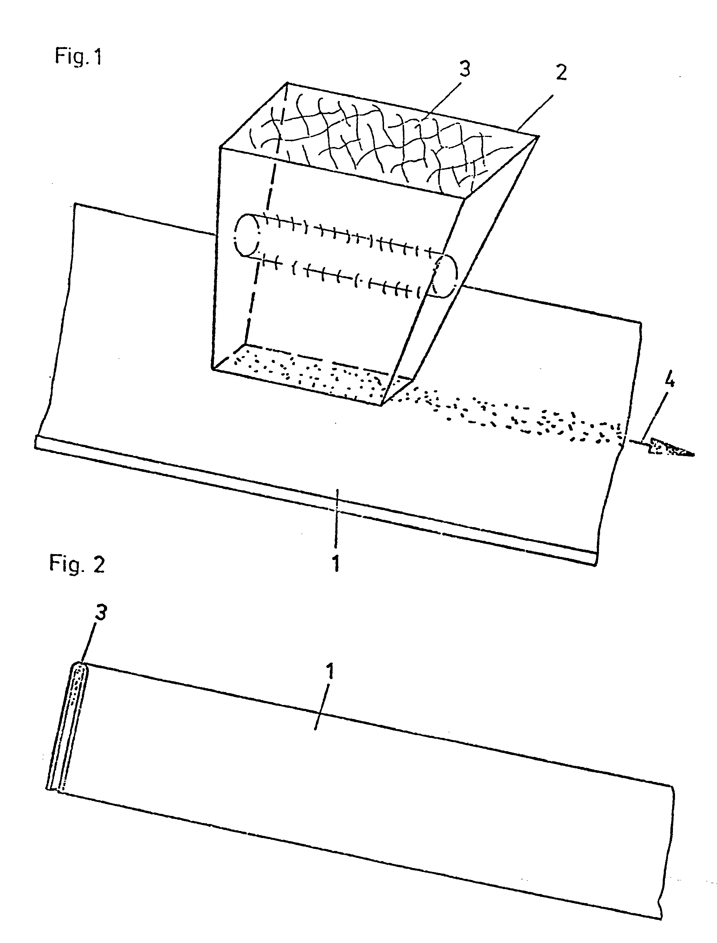

Über diesem Watteband 1 befindet sich gemäss Fig. 1 ein Reiss- oder Dosiergerät 2 zum Aufbringen der genannten Beimischung 3. Die Beimischung wird kontinuierlich während des Ablaufes des Wattebandes 1 in Pfeilrichtung 4, je nach dem, an welcher Stelle es gewünscht wird, in engen Streifen 5 in Fliessrichtung auf das Watteband 1 aufgestreut.

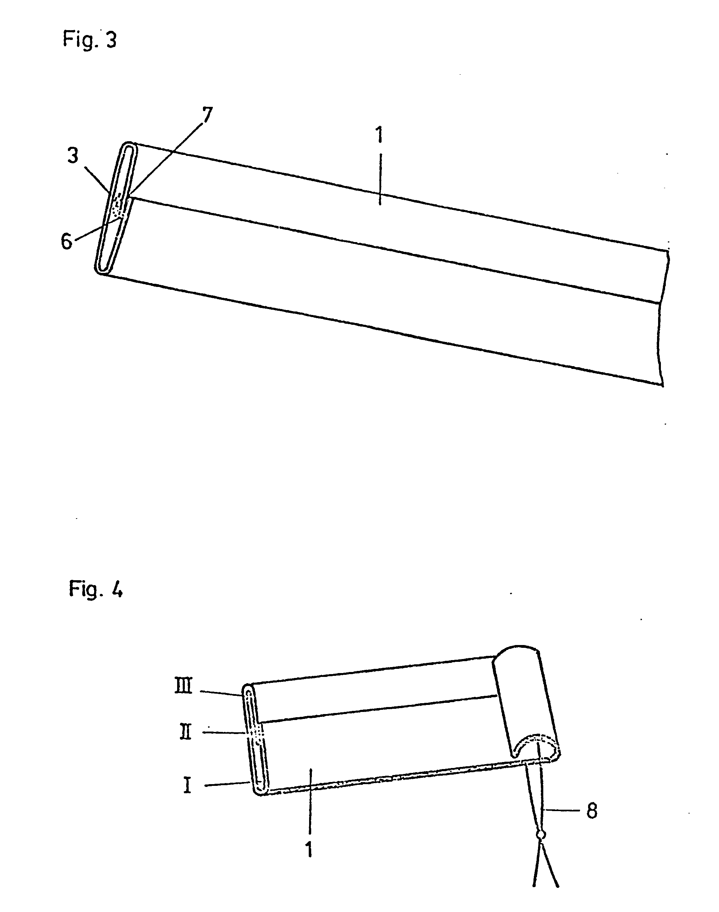

Nach dem Aufbringen der Beimischung 3 wird das Watteband 2 gemäss Fig. 2 auf sein Endformat gefaltet. Dabei wird die Beimischung 3 zweckmässig in den überlappenden Teil des Wattebandes eingeschlossen, um ein späteres Herausstreuen zu verhindern. Sehr günstig kann es auch sein, gemäss Fig. 3 eine Doppelfaltung vorzunehmen, in der Weise, dass beide Längsränder 6 und 7 des Wattebandes um 180° umgeklappt und aufeinander gelegt werden. Nach dem Falten wird beispielsweise über einen Waffel- oder Normalkalander erneut eine Pressung und anschliessend die Ablage in Rollen oder Hobbocks vorgenommen.

Auf diese Weise können an jeder beliebigen Stelle des Tampons besonders saugfähige und als Saugreserve dienende Zonen vorgesehen werden, die später beim Aufwickeln des Wattebandes sich mit Bandstärke (das heisst im Abstand der Dicke des Wattebandes) übereinander in Tampon schichten. Hierdurch ist es möglich, relativ hochverdichtete Saugzonen zu schaffen, die trotzdem wegen der besonderen Eigenschaften der fraglichen Beimischung, beispielsweise der CMC-Faser, zu keinen nennenswerten Erhöhungen des Pressdruckes in der Tampon-Maschine führen. Es besteht also keine Gefahr des Überpressens.

In Fig. 4 sind schematisch drei alternative Lagen I, II und III des aus der Beimischung 3 bestehenden Streifens 5 (Fig. 1) innerhalb eines gemäss Fig. 3 zusammengefalteten Wattebandes 1 dargestellt. Ausserdem geht aus Fig. 4 hervor, wie die Entfernungsschnur bzw. der Abziehfaden 8 beim Aufrollen des Tampons in diesen eingedreht werden kann.

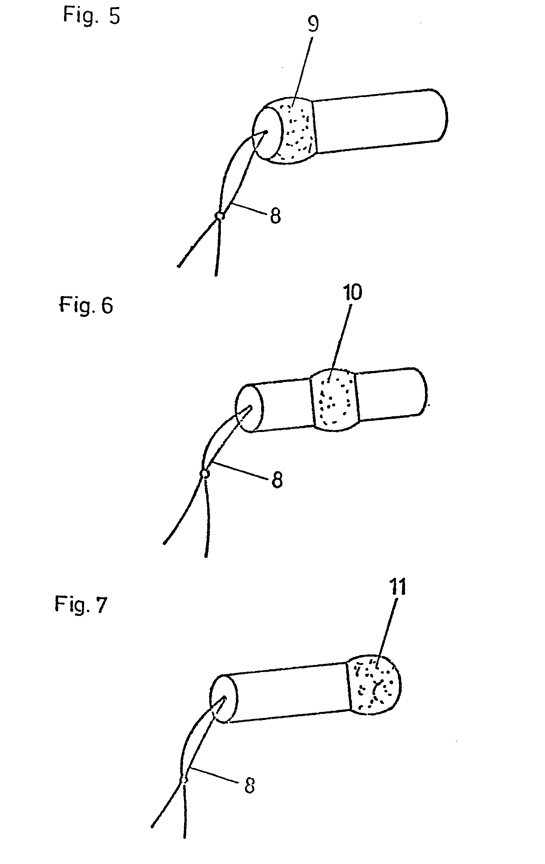

Die Fig. 5 bis 7 entsprechen der Reihe nach den Streifenlagen I, II und III von Fig. 5. Wenn also der Streifen, der erfindungsgemäss konzentriert innerhalb des Tampons angeordneten hydrophilen Beimischung die Lage I in Fig. 4 hat, entsteht gemäss Fig. 5 eine verdichtete Saugzone 9 am Ende des Tampons. Hat dagegen der Streifen die Lage II in Fig. 4, so liegt die verdichtete Saugzone 10 gemäss Fig. 6 in der Mitte des Tampons. Ist schliesslich der Streifen 5 der Beimischung an der Stelle III von Fig. 4 aufgestreut worden, so liegt die verdichtete Saugzone 11 am Kopf des Tampons.

Versuche haben ergeben, dass optimale Saugzonen mit Mengen von 2 bis 5% der die Hydrophilie verbessernden Beimischung, zum Beispiel Carboxymethylzellulose, erreicht werden können. Ähnliche prozentuale Anteile gelten für andere Stoffe, zum Beispiel Polymere oder geschäumte Polyurethane, mit besonderen Saugeigenschaften, die zur Verbesserung der Saugfähigkeit von Watte verwendet werden.

Liste der Bezugszeichen

1   = Watteband

2   = Reiss- oder Dosiergerät

3   = Beimischung

4   = Pfeilrichtung

5   = Streifen

6+7 = Ränder von 1

8   = Abziehfaden

9–11 = Saugzonen

I–III = Alternativlagen von 5

**Patentansprüche**

1. Menstruationstampon, insbesondere Wikkeltampon, aus beispielsweise aus Baumwolle oder Zellwolle bestehender Watte (1) mit einer hydrophilen Beimischung verstärkter Saugfähigkeit, insbesondere mit einer im Hinblick auf Hydrophilie modifizierten Faserbeimischung, dadurch gekennzeichnet, dass auf einem in bezug auf die Länge des Tampons relativ kurzen Längsabschnitt an dessen Ende (9), in dessen Mitte (10) oder an dessen Kopf (11) die Watte 2 bis 5% Beimischung enthält und die Watte der übrigen Bereiche des Tampons einen demgegenüber kleineren Anteil Beimischung enthält.

2. Verfahren zum Herstellen eines Wickeltampons nach Anspruch 1, dadurch gekennzeichnet, dass längs einer Linie parallel zur Längsrichtung eines – insbesondere bereits kalandrierten und gegebenenfalls mit einer Grundlast der genannten Beimischung (3) versetzten – Wattebandes (1) auf dieses ein gegenüber dem Watteband schmaler Streifen (5) der Beimischung aufgestreut wird, dass die Beimischung (3) durch Zusammenfalten der Ränder (6, 7) des Wattebandes (1) eingeschlossen wird, dass anschliessend ein Kalandrieren oder Pressen erfolgt und dass, gegebenenfalls nach einer Ablage in Rollen oder Hobbocks, die Tampons aufgewickelt werden, wobei die Streifen der Beimischung (3) mit Bandstärke übereinander geschichtet werden.

**Claims**

1. Menstruation tampon, particularly coiled tampon, of cotton wool (1) consisting for example of cotton or rayon with a hydrophilic admixture of increased absorbency, particularly with a fibrous admixture modified in respect of hydrophilia, characterised thereby, that the cotton wool contains 2 to 5% of admixture over a length section which is relatively short with respect to the length of the tampon and at the end (9), the middle (10) or the head (11) thereof and the cotton wool of the remaining regions of the tampon contain a proportion, which is smaller by comparison therewith, of the admixture.

2. Process for the production of a coiled tampon according to claim 1, characterised thereby, that along a line parallel to the longitudinal direction of a cotton wool tape (1), particularly an already calendered one and in a given case permeated by a basic charge of the named admixture (3), a strip (5), narrow by comparison with the cotton wool tape, of the admixture is scattered onto this, that the admixture (3) is enclosed by folding together the edges (6, 7) of the cotton wool tape (1), that a calendering or pressing takes place subsequently and that the tampons are coiled, in a given case after a deposit in rolls or canisters, wherein the strips of the admixture (3) are layered over one another in tape thickness.

**Revendications**

1. Tampon périodique, en particulier enroulé, en ouate (1) formée par exemple de coton ou de laine cellulosique et contenant un additif hydrophile à pouvoir absorbant renforcé, en particulier un additif fibreux modifié en vue de l'hydrophilie, caractérisé par le fait que sur une partie relativement petite de sa longueur, à son extrémité (9), au milieu (10) ou à sa tête (11), l'ouate contient 2 à 5% d'additif (3) et que l'ouate des autres régions du tampon contient, relativement à cela, une proportion plus petite d'additif.

2. Procédé de fabrication d'un tampon périodique enroulé selon la revendication 1, caractérisé par le fait que, le long d'une ligne parallèle à la direction longitudinale d'une bande d'ouate (1) – en particulier déjà calandrée et dans laquelle éventuellement a été additionnée une charge de base de l'additif mentionné (3) –, on répand sur cette bande une traînée (5) de l'additif améliorant l'hydrophilie, étroite par rapport à la largeur de la bande d'ouate, puis que l'on enferme cette traînée (5) d'additif (3) en repliant les bords (6, 7) de la bande d'ouate (1), qu'ensuite on opère un calandrage ou une compression sur cette bande repliée et qu'après avoir éventuellement mis cette dernière en rouleaux ou en bobines, on la reprend pour la rouler transversalement sur elle-même en superposant la traînée de l'additif (3) avec un espacement égal à l'épaisseur de la bande, jusqu'à atteindre la taille d'un tampon.

Fig. 1

3

2

4

1

Fig. 2

3

1

Fig. 3

Fig. 4

Fig. 5

Fig. 6

Fig. 7